# EUROPÄISCHE PATENTSCHRIFT

(11) **EP 3 337 616 B1**
(45) Veröffentlichungstag und Bekanntmachung des Hinweises auf die Patenterteilung: **08.05.2019**
(21) Anmeldenummer: 16777890.1
(22) Anmeldetag: 18.08.2016
(51) Int. Cl.: B01L 9/00

(54) **PIPETTENSPITZEN-AUFNAHMEBEHÄLTER SOWIE VERFAHREN ZU DESSEN BEREITSTELLUNG**
PIPETTE-TIP-ACCOMMODATING CONTAINER AND METHOD FOR PROVIDING THE SAME
RÉCIPIENT DE RÉCEPTION DE POINTES DE PIPETTES ET PROCÉDÉ DE FOURNITURE CORRESPONDANT

(30) Priorität: 21.08.2015 AT 507322015
(43) Veröffentlichungstag der Anmeldung: 27.06.2018
(73) Patentinhaber: Greiner Bio-One GmbH, 4550 Kremsmünster (AT)
(72) Erfinder: KOFLER, Georg, 4565 Inzersdorf im Kremstal (AT)
(74) Vertreter: Burger, Hannes
(86) Internationale Anmeldenummer: PCT/AT2016/060031
(87) Internationale Veröffentlichungsnummer: WO 2017/031514

(56) Entgegenhaltungen:
- WO-A1-2009/053434
- US-A- 6 019 225
- US-A1- 2008 240 999
- US-A1- 2012 328 489
- US-B1- 6 221 317

## Beschreibung

Die Erfindung betrifft einen Pipettenspitzen-Aufnahmebehälter zur Aufnahme einer Mehrzahl von Pipettenspitzen, wie dieser im Anspruch 1 beschrieben ist. Die Erfindung betrifft aber auch noch ein Verfahren zum Bereitstellen eines Pipettenspitzen-Aufnahmebehälters zur Aufnahme einer Mehrzahl von Pipettenspitzen, wie dies im Anspruch 16 beschrieben ist.

Aus der US 6,221,317 B1 ist eine gattungsgemäße Vorrichtung zum Bereitstellen von Pipettenspitzen bekannt geworden, bei welcher der Basiskörper die normierte Standfläche mit der festgelegten Längenabmessung und mit der im rechten Winkel dazu ausgerichteten und festgelegten Breitenabmessung aufweist. Zur Erzielung der normierten Standfläche ist der Basiskörper in seinem äußeren Umfangsbereich mit einer überwiegend doppelwandigen Wandstruktur ausgebildet, wobei hier die äußeren doppelten Wandteile die normierte Standfläche bilden. Im Inneren des Basiskörpers sind bis zu seiner offenen Oberseite reichende, einander schneidende Stege unter Ausbildung von dazwischen angeordneten Vertiefungen in einem vorbestimmten Raster angeordnet. Auf diesen Stegen ist eine Tragplatte mit darin angeordneten und zur Aufnahme der Pipettenspitzen dienenden Öffnungen abgestützt. Die Anzahl sowie die Rasteranordnung der Öffnungen in der Tragplatte wird in Abhängigkeit von der Größe und der Anzahl der auf der Tragplatte aufzunehmenden Pipettenspitzen sowie der festgelegten Norm (SBS-Standard) gewählt. Die Rasteranordnung der Stege im Basiskörper ermöglicht die Verwendung von zueinander unterschiedlichen Tragplatten bezüglich der Anzahl von aufzunehmenden Pipettenspitzen sowie deren Anordnung auf der Tragplatte. So kann stets der gleiche Basiskörper mit der normierten Standfläche zur Bereitstellung der Pipettenspitzen verwendet werden. Es ist dazu nur die für den jeweiligen Verwendungszweck entsprechende Tragplatte einzusetzen, um mittels des Basiskörpers die für eine automatisierte Probenverarbeitung notwendige Positionierung der Pipettenspitzen zu erzielen. Der Basiskörper der Bereitstellungsvorrichtung ist an seiner Oberseite noch mit einem aufsteckbaren Deckel verschließbar. Bei jeweils auf den Basiskörper aufgesetztem Deckel können auch mehrere gleichartig ausgebildete Bereitstellungsvorrichtung zu einem Stapel übereinander angeordnet werden. Dazu sind an der Oberseite des Deckels sowie an der Unterseite des Basiskörpers jeweils zusammenwirkende Formschlussstrukturen vorgesehen.

Die US 2014/0234182 A1 sowie die daraus hervorgegangene WO 2014/130679 A1 beschreiben einen Pipettenspitzen-Aufnahmebehälter zur Aufnahme einer Mehrzahl von Pipettenspitzen. Der Pipettenspitzen-Aufnahmebehälter umfasst eine Aufnahmeschale mit einem Boden und mit vom Boden aufragenden Seitenwänden. Die Seitenwände umgrenzen zumindest bereichsweise eine Aufnahmeöffnung sowie definieren gemeinsam mit dem Boden einen Aufnahmeraum. Weiters umfasst der Pipettenspitzen-Aufnahmebehälter einen Deckel mit einer Deckelwand und mit von der Deckelwand abstehenden Deckelseitenwänden, wobei die Deckelseitenwände gemeinsam mit der Deckelwand einen Deckelinnenraum definieren. In der Aufnahmeschale ist im Bereich ihrer Aufnahmeöffnung ein Träger mit darin angeordneten Aufnahmeöffnungen zur ausgerichteten Aufnahme der Pipettenspitzen angeordnet. Weiters ist die Aufnahmeschale im Bereich ihrer Seitenwände einschalig ausgebildet. In der Verschlussstellung des Deckels deckt dieser die Aufnahmeöffnung der Aufnahmeschale ab, wobei der Deckel abnehmbar an der Aufnahmeschale gehalten ist. Die Aufnahmeschale weist an ihrer Außenseite sowie im Bereich ihres Bodens Stützelemente auf, welche auf die vom Aufnahmeraum abgewendete Richtung vorspringen. Die Stützelemente dienen zur Bildung einer normierten Standfläche (footprint) für die Aufnahmeschale. Zusätzlich können im Bereich der Seitenwände sowie der Stützelemente Rippen angeordnet sein, welche auf die vom Aufnahmeraum abgewendete Richtung vorspringen und mit der Seitenwand sowie dem Stützelement verbunden sind. Nachteilig dabei ist, dass durch die zusätzlichen Stützelemente je Aufnahmeschale ein höherer Materialbedarf zur Herstellung notwendig ist und darüber hinaus auch noch für die manuelle Handhabung die Aufnahmeschale schwerer zu erfassen ist.

Der vorliegenden Erfindung liegt die Aufgabe zugrunde, die Nachteile des Standes der Technik zu überwinden und einen universell einsetzbaren Pipettenspitzen-Aufnahmebehälter zur Verfügung zu stellen, welcher einfach und kostengünstig mit geringem Rohmaterialbedarf herzustellen ist und sowohl für die manuelle Probenverarbeitung als auch die automatisierte Probenverarbeitung eingesetzt werden kann. Darüber hinaus sollen aber auch noch baugleich ausgebildete Aufnahmeschalen bis zu deren Komplettierung platzsparend transportiert und gelagert werden können.

Diese Aufgabe der Erfindung wird dadurch gelöst,
- dass der Deckel in seinem äußeren Umfangsbereich zumindest abschnittsweise eine gemäß der Norm SLAS 1-2004 (R2012) des American National Standards Instituts (ANSI) normierte Standfläche (footprint) mit einer Längenabmessung mit einem Wert von 127,76 mm ± 0,25 mm und mit einer im rechten Winkel dazu ausgerichteten Breitenabmessung mit einem Wert von 85,48 mm ± 0,25 mm definiert,
- dass am Deckel eine Positioniervorrichtung mit mehreren, insbesondere über den Umfang des Deckels, verteilt angeordneten Positionierelementen vorgesehen ist,
- dass der Deckel bei einer von der Aufnahmeschale abgenommenen Freigabestellung ein Adapterelement für die Aufnahmeschale bildet, und
- dass die Aufnahmeschale in der abgenommenen Freigabestellung des Deckels mit ihrem Boden an dem von der Aufnahmeschale abgenommenen Deckel zur Bildung einer Adapterstellung abgestützt ist, und in dieser Adapterstellung die Aufnahmeschale mittels der Positionierelemente relativ bezüglich der durch den Deckel definierten Standfläche (footprint) in einer Positionierstellung für eine automatisierte Probenverarbeitung positioniert ausgerichtet ist.

Der dadurch erzielte Vorteil liegt darin, dass so die Aufnahmeschale in einer einfachen, materialsparenden sowie für die manuelle Handhabung leicht zu erfassenden Ausbildung hergestellt werden kann. Dabei dient der Deckel des Pipettenspitzen-Aufnahmebehälters als Adapterelement zur Abstützung und präzisen Positionierung der Aufnahmeschale für die automatisierte Probenverarbeitung und es kann die Aufnahmeschale in ihren Abmessungen, insbesondere im Bereich ihres Bodens, unabhängig von den für die automatisierte Probenverarbeitung normierten Abmessungen ausgebildet werden und trotzdem für beide Arten der Probenverarbeitung eingesetzt werden. Dadurch kann bei der Ausbildung der Aufnahmeschale eine nicht unbeträchtliche Menge bzw. Masse an Werkstoff, insbesondere Kunststoff, eingespart werden. Weiters kann auch mit einfacher ausgebildeten Formwerkzeugen die Aufnahmeschale hergestellt werden. Durch den geringeren Materialeinsatz kann aber auch mit geringeren Taktzeiten die Herstellung erfolgen, wodurch ebenfalls Einsparungen erzielbar sind. Darüber hinaus kann so der Deckel in seiner Grundrissform einfach an die normierten Abmessungen der Standfläche angepasst werden. Somit können die Deckelseitenwände im Bereich der Deckelwand in deren äußeren Umfangsbereich nahezu geradlinig und ebenflächig ausgebildet werden. Auf zusätzliche Anschlagmittel oder dergleichen, kann somit verzichtet werden, um die normierten Abmessungen als Positionieranschläge einzuhalten und auszubilden.

Weiters wird damit der Deckel exakt auf die gemäß der Norm festgelegten Abmessungen abgestimmt und ausgebildet. Durch das Festlegen und Einhalten der normierten Abmessungen ist so lediglich der Deckel in seinen äußeren Abmessungen darauf abzustimmen und es kann die Aufnahmeschale einfacher und materialsparender ausgebildet werden. Bevorzugt können damit die Deckelseitenwände geradlinig bzw. ebenflächig ohne zusätzliche Vorsprünge oder dgl. für die exakte Einhaltung der normierten Standfläche ausgebildet werden.

Eine weitere Ausbildung sieht vor, dass die Positioniervorrichtung im Deckelinnenraum des Deckels sowie im Bereich seiner Deckelwand angeordnet oder ausgebildet ist. Damit kann der Deckelinnenraum des Deckels zur Aufnahme und Abstützung der Aufnahmeschale dienen. Weiters kann damit auch mit einem geringen Platzbedarf in der Adapterstellung erzielt werden, da die Deckelseitenwände seitlich der Aufnahmeschale von der Deckelwand aufragen.

Weiters kann es vorteilhaft sein, wenn die Positioniervorrichtung an der Deckelwand sowie an seiner vom Deckelinnenraum abgewendeten Seite angeordnet oder ausgebildet ist. Damit kann der Deckel eine Doppelfunktion erfüllen. Einerseits kann durch diesen das Adapterelement für die Aufnahmeschale ausgebildet werden und andererseits auch gleichzeitig die Positionierelemente für die Stapelbildung von mehreren Pipettenspitzen-Aufnahmebehältern bilden.

Eine andere Ausführungsform zeichnet sich dadurch aus, dass die Aufnahmeschale in deren Positionierstellung direkt an der Deckelwand abgestützt ist. Dadurch kann die Aufhahmeschale nur mit einer geringen zusätzlichen Überhöhung bezüglich ihrer eigenen Bauhöhe der automatisierten Probenverarbeitung bzw. der Entnahme von Pipettenspitzen zugeführt werden. Weiters kann damit aber auch die Sandfestigkeit der Aufnahmeschale im Deckel verbessert und ein unbeabsichtigtes Kippen vermieden werden.

Eine weitere mögliche Ausführungsform hat die Merkmale, dass die Aufnahmeschale jeweils in Übergangsbereichen zwischen den in Umfangsrichtung hintereinander angeordneten Seitenwänden an diesen sowie in deren dem Boden zugewendeten Umfangsbereich an den Positionierelementen abgestützt ist. Dadurch können die einzelnen Positionierelemente im Bereich des Deckelinnenraums mit einem relativ geringen Überstand über die Deckelwand ausgebildet werden und trotzdem eine ausreichende und sichere relative Positionierung zueinander erreicht werden.

Eine weitere Ausbildung sieht vor, dass am Deckel, insbesondere an dessen Deckelwand, für jeden der Übergangsbereiche der Aufnahmeschale zumindest zwei Positionierelemente vorgesehen sind. Damit kann die Aufnahmeschale in jedem ihrer Eck -bzw. Übergangsbereiche zwischen den einzelnen Seitenwänden exakt im Deckel positioniert werden. Durch diese Vielzahl der Positionierelemente kann so die Aufnahmeschale bereits beim Einsetzvorgang in den Deckelinnenraum besser geführt und nachfolgend exakt positioniert ausgerichtet und in dieser Position nahezu unverschieblich gehalten werden.

Eine weitere bevorzugte Ausführungsform ist dadurch gekennzeichnet, dass bei sich in der Positionierstellung befindlichen Aufnahmeschale diese zentrisch bezüglich der vom Deckel definierten Standfläche (footprint) ausgerichtet ist. Damit kann die Aufnahmeschale ohne vorheriger, gegenseitiger Ausrichtung einfach und unabhängig von der relativen Lage des Deckels in den Deckelinnenraum eingesetzt werden.

Weiters kann es vorteilhaft sein, wenn der Deckel mittels zumindest einer Schwenkanordnung an der Aufnahmeschale schwenkbar gelagert ist. Dadurch kann für die manuelle Probenentnahme der Deckel einfach aufgeschwenkt werden, um so einen Zugang zu den im Pipettenspitzen-Aufnahmebehälter bevorrateten oder aufgenommenen Pipettenspitzen zu ermöglichen. Weiters wird damit aber auch eine zusammengehörige Baueinheit aus Aufnahmeschale und Deckel gebildet.

Eine andere alternative Ausführungsform zeichnet sich dadurch aus, dass die Aufnahmeschale im Bereich ihrer Seitenwände einschalig ausgebildet ist. Durch die zumindest im Bereich der Seitenwände ausgebildete, einschalige Aufnahmeschale kann so eine hohe Materialeinsparung erzielt werden. Darüber hinaus können so aber auch einfacher ausgebildete Werkzeuge zur Bildung der Aufnahmeschale eingesetzt werden.

Eine weitere mögliche und gegebenenfalls alternative Ausführungsform hat die Merkmale, dass die Seitenwände der Aufnahmeschale im Axialschnitt gesehen jeweils ausgehend von der Aufnahmeöffnung hin in Richtung auf den Boden konisch verjüngend ausgebildet sind und dass die Aufnahmeschale im Bereich ihrer Aufnahmeöffnung eine lichte Querschnittsabmessung aufweist, welche größer ausgebildet ist als eine äußere Querschnittsabmessung der Aufnahmeschale im Bereich des Bodens. Damit kann die grundsätzliche Möglichkeit geschaffen werden, Aufnahmeschalen gleicher Bauart bis hin zum Fügen bzw. Aufsetzen des Deckels platzsparend ineinander stapeln zu können. Durch die Wahl der entsprechenden Öffnungsweite der Aufnahmeöffnung kann so das Ausmaß festgelegt werden, welche Stapelhöhe von gleichartig ineinander gestapelten Aufnahmeschalen erzielbar ist. Je größer die Differenz in den Querschnittsabmessungen ist, desto tiefer und umso platzsparender können mehrere Aufnahmeschalen ineinander gestapelt und so für den Transport sowie die Lagerhaltung mit einem geringeren Volumen das Auslangen gefunden werden.

Eine andere Ausführungsform zeichnet sich dadurch aus, dass an der Aufnahmeschale im Bereich ihres Aufnahmeraums zumindest ein erstes Stapelmittel angeordnet oder ausgebildet ist, und eine weitere baugleich ausgebildete Aufnahmeschale in den Aufnahmeraum einsetzbar und an dem zumindest einen ersten Stapelmittel abstützbar ist. Durch die gegenseitige Anschlagbegrenzung kann ein gegenseitiges Verklemmen von Aufnahmeschalen bei deren Stapelbildung verhindert werden.

Eine weitere bevorzugte Ausführungsform ist dadurch gekennzeichnet, dass der Boden der Aufnahmeschale zumindest bereichsweise ein weiteres Stapelmittel bildet. Dadurch kann mit geringstem Aufwand ein ineinander Stapeln von mehreren baugleich ausgebildeten Aufnahmeschalen ermöglicht werden.

Eine weitere Ausbildung sieht vor, dass in der Aufnahmeschale im Bereich ihrer Aufnahmeöffnung ein Träger mit darin angeordneten Zentrieraufnahmen zur ausgerichteten Aufnahme der Pipettenspitzen angeordnet oder ausgebildet ist. Dadurch wird die Möglichkeit geschaffen, die Pipettenspitzen in entsprechender exakter Ausrichtung innerhalb des Pipettenspitzen-Aufnahmebehälters bereitstellen zu können.

Eine andere Ausführungsform zeichnet sich dadurch aus, dass der Deckel in seiner Verschlussstellung mittels zumindest einer Verschlussvorrichtung an der Aufnahmeschale arretiert gehalten ist, wobei die zumindest eine Verschlussvorrichtung am Deckel ein erstes Verschlusselement und an der Aufnahmeschale ein damit zusammenwirkendes zweites Verschlusselement umfasst. Durch die miteinander in Eingriff stehenden Verschlusselemente kann ein unbeabsichtigtes Öffnen des Deckels verhindert werden. Damit kann eine geschützte Aufbewahrung der Pipettenspitzen erzielt werden.

Eine weitere bevorzugte Ausfuhrungsform ist dadurch gekennzeichnet, dass das am Deckel, insbesondere an zumindest einer seiner Deckelseitenwände, angeordnete erste Verschlusselement lappenförmig ausgebildet ist und schwenkbar mit dem Deckel verbunden ist, und dass bei sich in der Positionierstellung befindlicher Aufnahmeschale das erste Verschlusselement in den Deckelinnenraum eingeschwenkt ist und an der unmittelbar benachbart angeordneten Seitenwand der Aufnahmeschale anliegt. Durch die lappenförmige Ausbildung des Verschlusselements kann dieses in mehreren Funktionen eingesetzt werden. In der normalen Gebrauchslage dient das erste Verschlusselement dazu, den Deckel in seiner Verschlussstellung arretiert an der Aufnahmeschale zu halten. In einer weiteren Verwendung dient das erste Verschlusselement noch dazu, in den zwischen den Seitenwänden der Aufnahmeschale und den Deckelseitenwänden ausgebildeten Zwischenraum hinein verschwenkt zu werden. Durch dieses hinein Verschwenken und Hineinragen in den Zwischenraum kann so eine Druckkraft aufgrund der Rückstellwirkung des lappenförmigen Verschlusselements auf die Aufnahmeschale ausgeübt werden. Weiters können damit über den Außenumfang des Deckels seitlich darüber hinausstehende Teile am Deckel vermieden werden, welche bei der automatisierten Probenverarbeitung störend oder sogar hindernd sein könnten.

Eine andere Ausführungsform zeichnet sich dadurch aus, dass bei sich in der Positionierstellung befindlichen Aufnahmeschale unmittelbar einander benachbart angeordnete Seitenwände der Aufnahmeschale und Deckelseitenwände des Deckels in Umfangsrichtung gesehen jeweils im Wesentlichen parallel zueinander verlaufend ausgerichtet sind. Dadurch kann für den Benutzer eine einfache optische Kontrolle bereitgestellt werden, um so die exakte Positionierung der Aufnahmeschale im Deckel einfach kontrollieren zu können.

Die Aufgabe der Erfindung kann aber unabhängig davon auch durch ein Verfahren zum Bereitstellen eines Pipettenspitzen-Aufnahmebehälters zur Aufnahme einer Mehrzahl von Pipettenspitzen gemäß den im Anspruch 16 angegebenen Merkmalen gelöst werden. Die aus der Merkmalskombination dieses Anspruches erzielten Vorteile liegen darin, dass dadurch die Aufnahmeschale in einer einfachen, materialsparenden sowie für die manuelle Handhabung leicht zu erfassende Ausbildung hergestellt werden kann. Dabei dient der Deckel des Pipettenspitzen-Aufhahmebehälters als Adapterelement zur Aufnahme und präzisen Positionierung der Aufnahmeschale für die automatisierte Probenverarbeitung und es kann die Aufnahmeschale in ihren Abmessungen, insbesondere im Bereich ihres Bodens, unabhängig von den für die automatisierte Probenverarbeitung normierten Abmessungen ausgebildet werden und trotzdem für beide Arten der Probenverarbeitung eingesetzt werden. Dadurch kann bei der Ausbildung der Aufnahmeschale eine nicht unbeträchtliche Menge bzw. Masse an Werkstoff, insbesondere Kunststoff, eingespart werden. Weiters kann auch mit einfacher ausgebildeten Formwerkzeugen die Aufnahmeschale hergestellt werden. Durch den geringeren Materialeinsatz kann aber auch mit geringeren Taktzeiten die Herstellung erfolgen, wodurch ebenfalls Einsparungen erzielbar sind.

Eine weitere vorteilhafte Vorgehensweise ist dadurch gekennzeichnet, dass die Aufnahmeschale in deren Positionierstellung im Deckel zentrisch bezüglich der vom Deckel definierten Standfläche (footprint) ausgerichtet wird. Damit kann die Aufnahmeschale ohne vorheriger, gegenseitiger Ausrichtung einfach und unabhängig von der relativen Lage des Deckels in den Deckelinnenraum eingesetzt werden.

Schließlich ist auch eine Verfahrensvariante vorteilhaft, bei welcher die Aufnahmeschale in deren Positionierstellung im Deckel zusätzlich mit einer in etwa parallel bezüglich der Deckelwand ausgerichteten Stellkraft auf zumindest eine ihrer Seitenwände wirkend beaufschlagt wird und die Aufnahmeschale in Wirkungsrichtung der Stellkraft von dieser gegen zumindest einzelne der Positionierelemente gedrückt wird. Damit kann vom ersten Verschlusselement in dessen eingeschwenkter Position in den zwischen den Seitenwänden der Aufnahmeschale und den Deckelseitenwänden ausgebildeten Zwischenraum eine gerichtete Andrückkraft auf die Aufnahmeschale und weiters auf die gegenüberliegend angeordneten Positionierelemente ausgeübt werden.

Zum besseren Verständnis der Erfindung wird diese anhand der nachfolgenden Figuren näher erläutert.

Es zeigen jeweils in stark vereinfachter, schematischer Darstellung:
- Fig. 1: einen Pipettenspitzen-Aufnahmebehälter in der Verschlussstellung des Deckels an der Aufhahmeschale, in schaubildlicher Darstellung;
- Fig. 2: den Pipettenspitzen-Aufnahmebehälter nach Fig. 1, jedoch in der Freigabestellung des Deckels, in schaubildlicher Darstellung;
- Fig. 3: den Pipettenspitzen-Aufnahmebehälter nach den Fig. 1 und 2, jedoch bei von der Aufnahmeschale abgenommenem Deckel und der in den Deckel eingesetzten Positionierstellung der Aufnahmeschale, in schaubildlicher Darstellung;
- Fig. 4: den Deckel des Pipettenspitzen-Aufnahmebehälters in Ansicht auf seinen Deckelinnenraum, mit angedeutetem Umriss der Aufnahmeschale;
- Fig. 5: einen Teilausschnitt des Deckels und der darin aufgenommenen Aufnahmeschale im Bereich der Positioniervorrichtung, in Seitenansicht geschnitten gemäß den Linien V-V in Fig. 4;
- Fig. 6: einen anderen Teilausschnitt des Deckels und der darin aufgenommenen Aufnahmeschale mit einem an einer Seitenwand der Aufnahmeschale abgestützten Verschlusselement, in Seitenansicht geschnitten gemäß den Linien VI-VI in Fig. 3;
- Fig. 7: einen Teilausschnitt von baugleich ausgebildeten Aufhahmeschalen, in einer ineinander gestapelten Position, in Seitenansicht geschnitten;
- Fig. 8: einen Teilausschnitt einer weiteren möglichen Anordnung der Aufnahmeschale und des Deckels als Adapterelement, in Seitenansicht geschnitten.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

Der Begriff "insbesondere" wird nachfolgend so verstanden, dass es sich dabei um eine mögliche speziellere Ausbildung oder nähere Spezifizierung eines Gegenstands oder eines Verfahrensschritts handeln kann, aber nicht unbedingt eine zwingende, bevorzugte Ausführungsform desselben oder eine Vorgehensweise darstellen muss.

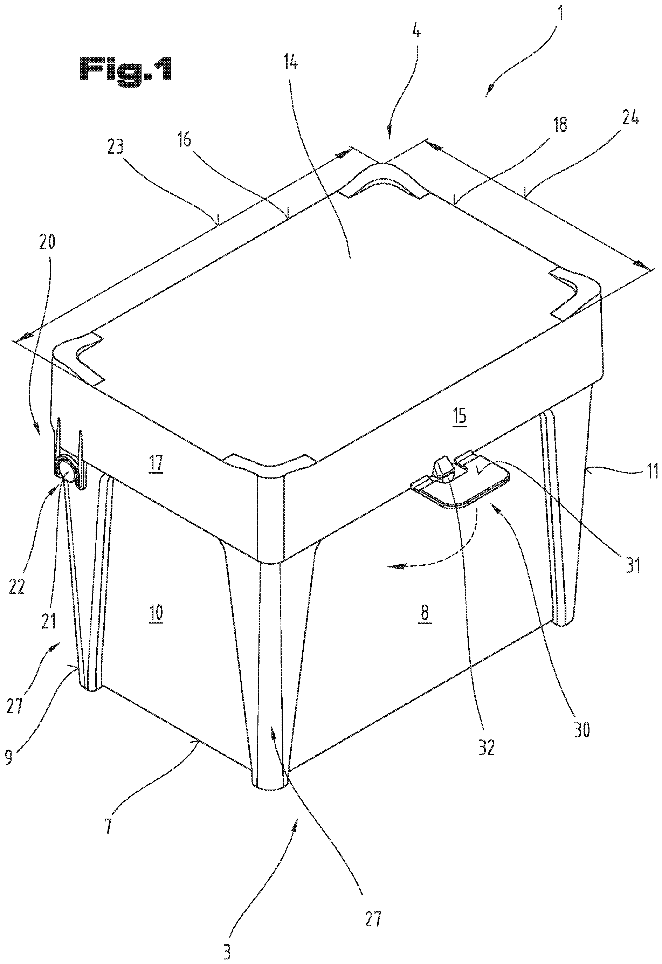

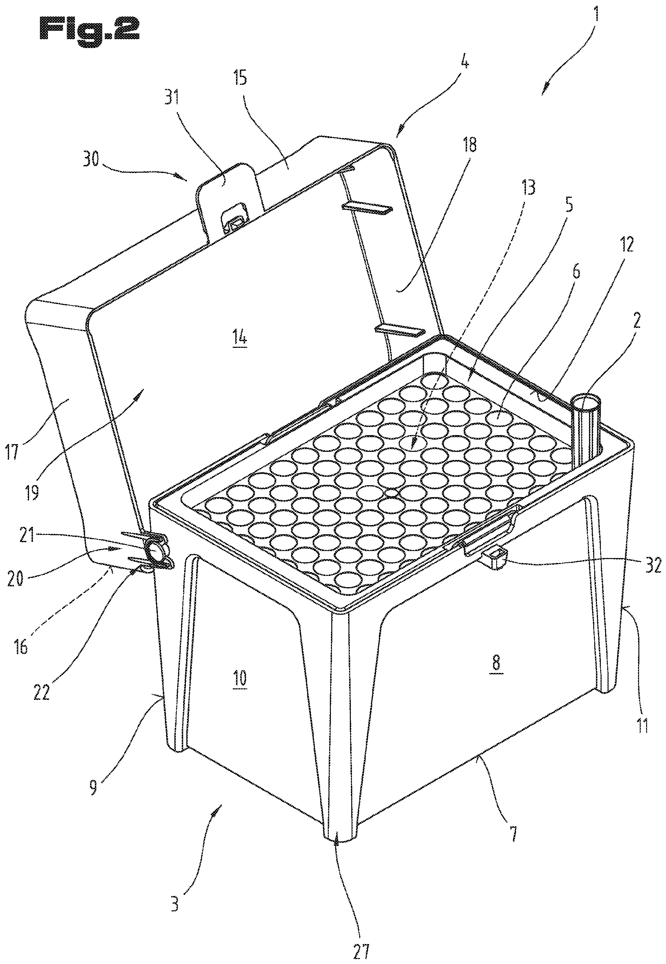

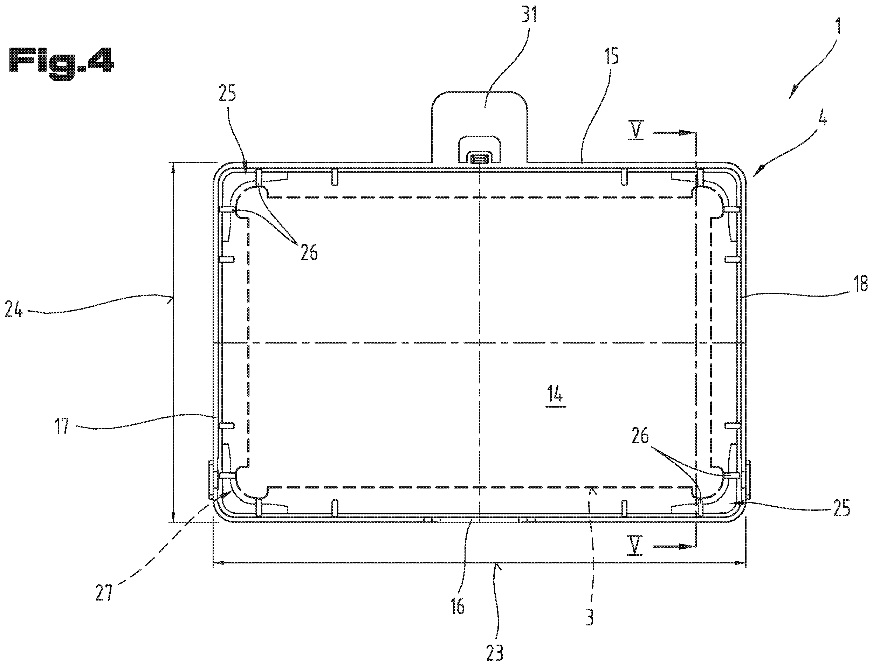

In den Fig. 1 bis 7 ist ein Pipettenspitzen-Aufnahmebehälter 1 zur Aufnahme einer Mehrzahl von Pipettenspitzen 2 gezeigt, welcher eine Aufnahmeschale 3, einen Deckel 4 und einen Träger 5 mit darin angeordneten oder ausgebildeten Zentrieraufnahmen 6 für die Pipettenspitzen 2 umfasst. Die zueinander Anordnung der Mehrzahl an Zentrieraufnahmen 6 ist zumeist genormt. Die Längs- und Querabstände zwischen Mittelachsen der Zentrieraufnahmen 6 betragen bevorzugt jeweils 9,00 mm, wobei diese Längs- und Querabstände ebenfalls in Übereinstimmung mit der entsprechenden Norm zu wählen sind. Die Zentrieraufnahmen 6 dienen zur ausgerichteten Aufnahme der Pipettenspitzen 2, wie dies bei derartigen Pipettenspitzen-Aufnahmebehältern 1 hinlänglich bekannt ist.

Die Aufnahmeschale 3 umfasst ihrerseits einen Boden 7 sowie jeweils vom Boden aufragend ausgerichtete Seitenwände 8 bis 11.Die Seitenwände 8 bis 11 umgrenzen zumindest bereichsweise an ihren von Boden 7 distanzierten Enden eine Aufnahmeöffnung 12 und definieren gemeinsam mit dem Boden 7 einen Aufnahmeraum 13.

Der Deckel 4 umfasst bei diesem Ausführungsbeispiel eine Deckelwand 14 sowie im Umfangsbereich derselben von dieser abstehende Deckelseitenwände 15 bis 18. Die Deckelseitenwände 15 bis 18 definieren gemeinsam mit der Deckelwand 14 einen Deckelinnenraum 19. An der Oberseite bzw. Außenseite des Deckels 4 sind an dessen äußerem Umfang jeweils in den Eckbereichen Positionierhilfen angeordnet, welche dazu dienen, den Boden einer bevorzugt baugleichen Aufnahmeschale 3 zur Stapelbildung mehrerer verschlossener Pipettenspitzen-Aufnahmebehälter 1 übereinander gegen Verrutschen zu halten. Die Positionierhilfen sind hier nicht umlaufend über den gesamten Umfang des Deckels 4 sondern nur in den Eckbereichen vorgesehen.

In der in der Fig. 1 dargestellten Verschlussstellung deckt der Deckel 4 die Aufnahmeöffnung 12 der Aufnahmeschale 3 ab. Weiters ist der Deckel 4 abnehmbar an der Aufnahmeschale 3 gehalten. Dies kann entweder durch eine reine Aufsteckverbindung erfolgen, wobei die Deckelseitenwände 15 bis 18 zumindest bereichsweise die Seitenwände 8 bis llder Aufnahmeschale 3 außenseitig übergreifen können. Bei diesem Ausführungsbeispiel ist gezeigt, dass der Deckel 4 mittels zumindest einer Schwenkanordnung 20 an der Aufnahmeschale 3 schwenkbar gelagert ist. Dazu sind im Bereich der beiden schmäleren bzw. kürzeren Seitenwände 10,11 jeweils Schwenkzapfen 21 angeordnet, insbesondere einstückig daran angeformt. Am Deckel 4, insbesondere den hier ebenfalls kürzer ausgebildeten Deckelseitenwänden 17 und 18, ist jeweils ein Schwenkauge 22 angeordnet, welches am besten aus einer Zusammenschau der Fig. 1 bis 3 zu ersehen ist. Das oder die Schwenkaugen 22 sind derart ausgebildet, dass einerseits ein Schwenken des Deckels 4 um den oder die Schwenkzapfen 21 möglich ist und der Deckel 4 durch elastische Verformung des oder der Schwenkaugen 22 vom Schwenkzapfen 21 bzw. den Schwenkzapfen 21 abnehmbar ist. Die Abnahme und die weitere Verwendung des Deckels 4 wird nachfolgend noch näher beschrieben.

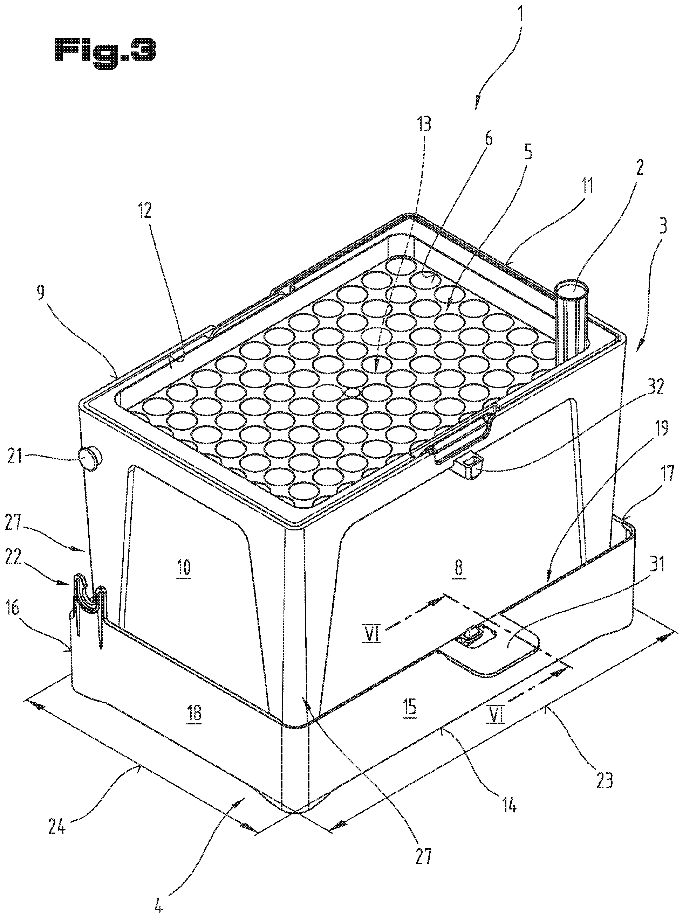

Wie nun besser aus den Darstellungen der Fig. 3 bis 5 zu ersehen ist, ist der Deckel 4 als Adapterelement für die Aufnahmeschale 3 ausgebildet. Unter einem Adapterelement wird hier verstanden, dass der Deckel 4 in seinem äußeren Umfangsbereich zumindest abschnittsweise eine normierte Standfläche mit einer Längenabmessung 23 und eine im rechten Winkel dazu ausgerichteten Breitenabmessung 24 definiert. Die normierte Standfläche kann auch als sogenannter "footprint" bezeichnet werden. In den unterschiedlichsten Labors sind für eine automatisierte Probenverarbeitung normierte Abmessungen der Standfläche eingeführt worden, um so mit automatisiert arbeitenden Anlagen die Pipettenspitzen-Aufnahmebehälter 1 unterschiedlicher Bauarten, wie diese von verschiedenen Herstellern am Markt befindlich sind, aufnehmen und verarbeiten zu können. Unter der automatisierten Probenverarbeitung wird hier allgemein verstanden, dass Pipettenspitzen 2 in der Anlage in eine exakt festgelegte Position verbracht werden und in dieser für die automatische Entnahme von einzelnen oder mehreren Pipettenspitzen 2 in der positionierten Stellung bzw. Position verbleiben. Da die Pipettenspitzen 2 in der Aufnahmeschale 3 selbst in einem vorgegebenen Rastermaß aufgenommen sind, ist die Aufnahmeschale 3 in die exakt festgelegte Position zu verbringen. Bei dieser Ausbildung dient nun der Deckel 4 dazu, einerseits die für die Positionierung normierte Standfläche zu bilden und andererseits die Aufnahmeschale 3 derart positioniert aufnehmen zu können, dass die Pipettenspitzen 2 bezüglich der normierten Standfläche dazu angeordnet sind.

Um eine gegenseitige positionierte Ausrichtung der Aufnahmeschale 3 im Deckel 4 gewährleisten zu können, ist im Bereich der Deckelwand 14 des Deckels 4 eine Positioniervorrichtung 25 vorgesehen. Die Positioniervorrichtung 25 kann ihrerseits mehrere, insbesondere über den Umfang des Deckels 4 verteilt angeordnete, Positionierelemente 26 umfassen. Bei diesem hier gezeigten Ausführungsbeispiel sind die Positionierelemente 26 dem äußeren Umfangsrand des Bodens 7 bzw. der Seitenwände 8 bis 11 zugeordnet oder wirken mit diesem oder diesen zusammen.

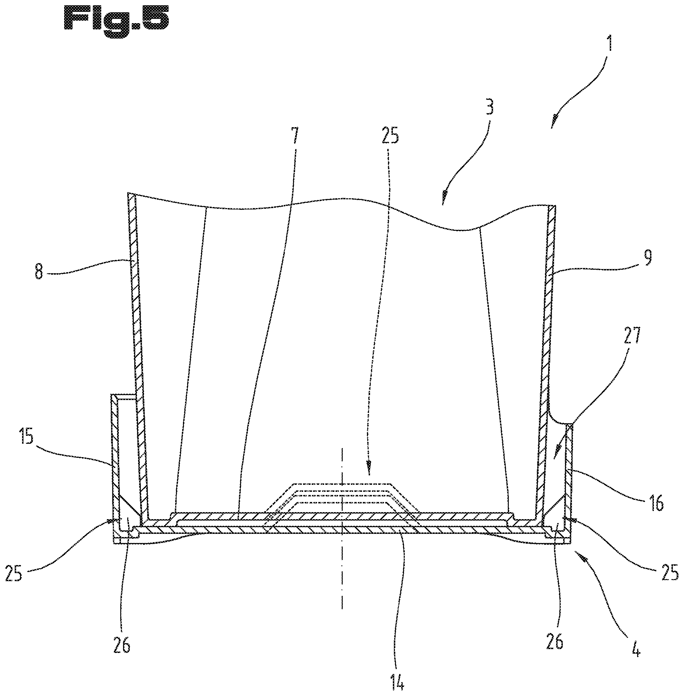

Es wäre aber auch unabhängig davon noch möglich, an der Deckelwand 14 auf seiner in der Positionierstellung dem Boden 7 zugewendeten Seite mehrere Positionierelemente 26 vorzusehen, welche über die Deckelwand 14 vorragen und jeweils in eine im Boden 7 angeordnete oder ausgebildete Zentrieröffnung in der Positionierstellung eingreifen. Diese Ausbildung ist vereinfacht im Bodenbereich der Fig. 5 in strichlierten Linien angedeutet.

Wird der Deckel 4 als Adapterelement für die Aufnahmeschale 3 eingesetzt, ist der Deckel 4 von der Aufnahmeschale 3 in eine Freigabestellung zu verbringen. Damit wird es möglich die Aufnahmeschale 3 mit ihrem Boden 7 am Deckel 4 abzustellen. Bei diesem Ausführungsbeispiel ist die Aufnahmeschale 3 in den Deckelinnenraum 19 des Deckels 4 einzusetzen. Dabei kann auch von einer Aufnahmestellung der Aufnahmeschale 3 im Deckel 4 gesprochen werden. Durch das Vorsehen der Positionierelemente 26 wird die Aufnahmeschale 3, relativ bezüglich der durch den Deckel 4 definierten Standfläche (footprint), in einer sogenannten Positionierstellung für die automatisierte Probenverarbeitung positioniert ausgerichtet. Dies bedeutet, dass nicht durch die Aufnahmeschale 3 die normierte Standfläche definiert bzw. festgelegt wird, sondern der Deckel 4 für die Aufnahmeschale 3 als Adapterelement dient. Damit wird die Möglichkeit geschaffen, die Aufnahmeschale 3 an die jeweiligen Erfordernisse und Abmessungen unabhängig von der normierten Standfläche ausbilden zu können. In diesem Fall wird für die Aufnahmeschale 3 erst in Kombination mit dem Deckel 4 als Adapterelement die normierte Standfläche für den Pipettenspitzen- Aufnahmebehälter 1 ausgebildet.

Das Ausmaß bzw. die Größe der normierten Standfläche (footprint) ist durch das "American National Standards Institut (ANSI)" in der Norm SLAS 1-2004 (R2012) festgelegt.

Gemäß dieser Norm zur Festlegung bzw. dem Ausmaß der normierten Standfläche (footprint) weist die Längenabmessung 23 einen Wert von 127,76 mm ± 0,25 mm auf. Die Breitenabmessung 24 weist ihrerseits einen Wert von 85,48 mm ± 0,25 mm auf.

Befindet sich die Aufnahmeschale 3 in deren Positionierstellung z.B. innerhalb des Deckels 4, kann diese direkt an der Deckelwand 14 abgestützt sein. Damit kann der Boden 7 der Aufnahmeschale 3 direkt an der Innenseite der Deckelwand 14 aufliegen.

Unmittelbar in Umfangsrichtung hintereinander angeordnete Seitenwände 8 bis 11 bilden jeweils zwischen diesen Übergangsbereiche 27 bzw. Eckbereiche aus. Im vorliegenden Ausführungsbeispiel ragen die Übergangsbereiche 27 in den durch die Seitenwände 8 bis 11 ausgebildeten Eckbereichen jeweils nach außen vor. Die Aufnahmeschale 3 ist jeweils in ihren Übergangsbereichen 27 sowie in deren dem Boden 7 zugewendeten Umfangsbereich zumindest abschnittsweise an den Positionierelementen 26 abgestützt. Bevorzugt sind am oder im Deckel 4 für jeden der Übergangsbereiche 27 der Aufnahmeschale 3 jeweils zwei Positionierelemente 26 vorgesehen. Damit kann jeder der Übergangsbereiche 27 - also die Aufnahmeschale 3 - in jedem ihrer Eckbereiche zwischen den Seitenwänden 8 bis 11 eindeutig positioniert werden. Die Positionierelemente 26 sind bevorzugt stegförmig oder rippenförmig ausgebildet. Die jeweils einem Übergangsbereich 27 bzw. Eckbereich zugeordneten Positionierelemente 26 weisen zueinander eine Ausrichtung unter einem Winkel von 90° auf.

Befindet sich die Aufnahmeschale 3 in deren Positionierstellung in Deckel 4, sind jeweils unmittelbar einander benachbart angeordnete Seitenwände 8 bis 11 der Aufnahmeschale 3 und Deckelseitenwände 15 bis 18 des Deckels 4 in Umfangsrichtung gesehen im Wesentlichen parallel zu einander verlaufend ausgerichtet.

In der Positionierstellung der Aufnahmeschale 3 ist diese zentrisch bezüglich der vom Deckel 4 definierten Standfläche ausgerichtet. Unter zentrisch wird hier verstanden, dass in der Positionierstellung das Zentrum der Aufnahmeschale 3 bezüglich des Zentrums des Deckels 4 deckungsgleich angeordnet ist. Bei entsprechender Anordnung und Ausrichtung der Zentrieraufnahmen 6 im Träger 5 sind damit auch die Pipettenspitzen 2 in einer eindeutigen relativen Position bezüglich der definierten Standfläche ausgerichtet und angeordnet.

Weiters ist bei diesem Ausführungsbeispiel gezeigt, dass die Aufnahmeschale 3 zumindest im Bereich ihrer Seitenwände 8 bis 11 einschalig ausgebildet ist. Dadurch kann mit einem geringeren Materialbedarf das Auslangen gefunden werden.

Zur Abstützung des Trägers 5 im Bereich der Aufnahmeöffnung 12 der Aufnahmeschale 3 können Teilabschnitte der Seitenwände 8 bis 11 in Richtung auf den Aufnahmeraum 13 hinein versetzt sein. Durch dieses Hineinversetzen kann so spritzgusstechnisch eine einfachere Ausbildung der Aufnahmeschale 3 erzielt werden und trotzdem die nach innen ragenden Teilabschnitte der Seitenwände 8 bis 11 eine Auflage bzw. Abstützung für den Träger 5 ausbilden.

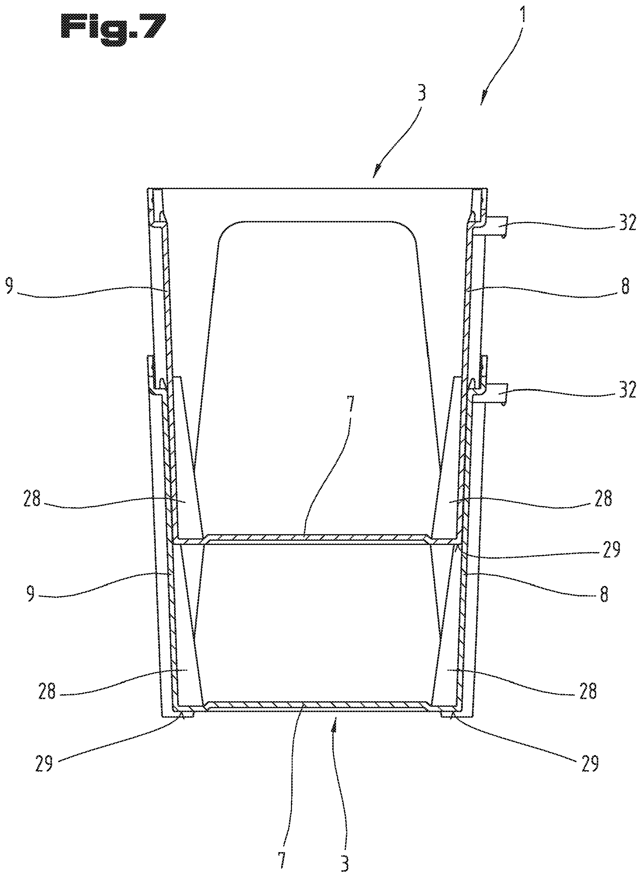

Weiters können die Seitenwände 8 bis 11 der Aufnahmeschale 3 im Axialschnitt gesehen, jeweils ausgehend von der Aufnahmeöffnung 12 hin in Richtung auf den Boden 7 konisch verjüngend ausgebildet sein. Damit kann nicht nur die Entformung der Aufnahmeschale 3 erleichtert werden, sondern bei entsprechender Wahl und Größe der konischen Verjüngung auch ein ineinander Stapeln baugleich ausgebildeter Aufnahmeschalen 3 ermöglicht werden. Dazu ist eine lichte Querschnittsabmessung der Aufnahmeschale 3 im Bereich ihrer Aufnahmeöffnung 12 größer auszubilden als eine äußere Querschnittsabmessung der Aufnahmeschale 3 im Bereich des Bodens 7. Dadurch wird es möglich, dass baugleich ausgebildete Aufnahmeschalen 3 ineinander gestapelt werden können, wie dies aus der Darstellung der Fig. 7 zu ersehen ist.

Um ein vordefiniertes Stapelmaß und somit das Ausmaß des Ineinandergreifens baugleicher Aufnahmeschalen 3 festlegen zu können, kann an der Aufnahmeschale 3 im Bereich ihres Aufnahmeraumes 13 zumindest ein erstes Stapelmittel 28 angeordnet oder ausgebildet sein. An diesem zumindest einen ersten Stapelmittel 28 ist eine weitere baugleich ausgebildete Aufnahmeschale 3 daran abstützbar. Im vorliegenden Ausführungsbeispiel kann beispielsweise der Boden 7 der Aufnahmeschale 3 zumindest bereichsweise ein weiteres Stapelmittel 29 ausbilden.

Wie nun besser aus der Fig. 1 zu ersehen ist, kann der Deckel 4 in seiner Verschlussstellung mittels zumindest einer Verschlussvorrichtung 30 an der Aufnahmeschale 3 arretiert gehalten sein. Die hier dargestellte zumindest eine Verschlussvorrichtung 30 kann am Deckel 4 ein erstes Verschlusselement 31 und an der Aufnahmeschale 3 ein damit zusammenwirkendes zweites Verschlusselement 32 umfassen.

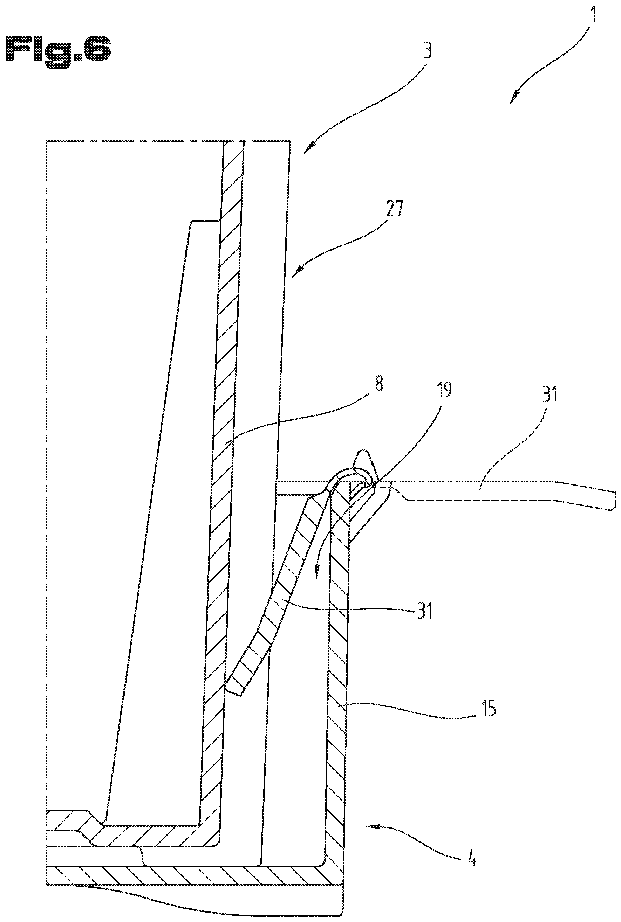

In der Fig. 1 ist die original unverformte Stellung des ersten Verschlusselements 31 gezeigt, wobei sich die beiden Verschlusselemente 31, 32 in einer noch nicht miteinander in Eingriff stehenden Arretier- bzw. Verschlussstellung befinden. Im vorliegenden Ausführungsbeispiel ist gezeigt, dass das erste Verschlusselement 31 lappenförmig ausgebildet ist und im Anschlussbereich an den Deckel 4 eine Ausnehmung bzw. Durchsetzung zur Aufnahme des zweiten Verschlusselements 32 aufweist. Damit kann das erste Verschlusselement 31 mit seiner Ausnehmung das zweite Verschlusselement 32 hintergreifen. Weiters ist das zumindest eine erste Verschlusselement 31 schwenkbar mit dem Deckel 4 verbunden. Dadurch wird es möglich, wie dies in der Fig. 6 dargestellt ist, dass bei sich in der Positionierstellung befindlicher Aufnahmeschale 3 das erste Verschlusselement 31 in den Deckelinnenraum 19 hinein geschwenkt ist und in Richtung auf die Deckelwand 14 ragt. In dieser Stellung liegt dann das erste Verschlusselement 31 an der jeweils dieser unmittelbar benachbart angeordneten Seitenwand 8 bis 11 der Aufnahmeschale 3 daran an. Die unverformte Originalstellung des ersten Verschlusselements 31 ist in strichlierten Linien dargestellt, wobei die umgeformte Position in Voll-Linien gezeigt ist.

Durch dieses nach innen Hineinschwenken des lappenförmig ausgebildeten ersten Verschlusselements 31 kann die Aufnahmeschale 3 in der Positionierstellung im Deckel 4 zusätzlich mit einer in etwa parallel bezüglich der Deckelwand 14 ausgerichteten Stellkraft beaufschlagt werden, welche auf die jeweils dieser zugewendete Seitenwand 8 bis 11 der Aufnahmeschale 3 einwirkt. Durch die aufgebaute Stellkraft wird die Aufnahmeschale 3 in Wirkungsrichtung derselben gegen zumindest einzelne der Positionierelemente 26 auf der jeweils gegenüber liegenden Seite gedrückt. Damit kann eine zusätzliche Halte- bzw. Fixierwirkung zwischen der Aufnahmeschale 3 und dem Deckel 4 in deren Positionierstellung erzielt werden.

Das Verfahren zum Bereitstellen des Pipettenspitzen-Aufnahmebehälters 1 kann, wie nachfolgend kurz beschrieben, folgende Schritte umfassen. Die einzelnen Pipettenspitzen 2 werden in den unterschiedlichsten Größen hergestellt und jeweils gleichartige derselben auf den zuvor beschriebenen Trägern 5 positioniert aufgenommen. Zumeist werden mehrere Träger 5 mit den jeweiligen Pipettenspitzen 2 zu einem Verbrauchsstapel zusammengestellt und je nach Anzahl und Größe in einer Verbrauchseinheit bereitgestellt.

Der Pipettenspitzen-Aufnahmebehälter 1 dient nun dazu, einen der Träger 5 im Bereich seiner Aufnahmeöffnung 12 der Aufnahmeschale 3 aufzunehmen und dort positioniert zu haltern. Für die Bereitstellung der Pipettenspitzen-Aufnahmebehälter 1 sind unterschiedliche Möglichkeiten denkbar. So könnte z.B. nur die Aufnahmeschale 3 mit dem darauf gehaltenen Deckel 4, jedoch ohne dem darin aufgenommenen Träger 5 und damit auch ohne den Pipettenspitzen 2 an den Anwender oder Benutzer geliefert werden. Dieser kann dann je nach Bedarf aus einer der Verbrauchseinheiten einen Träger 5 samt den darauf gehaltenen Pipettenspitzen 2 entnehmen und in die geöffnete Aufnahmeschale 3 einsetzen.

Es wäre aber auch noch möglich, dass der Pipettenspitzen-Aufnahmebehälter 1 samt dem darin aufgenommenen Träger 5 und mit den dort gehaltenen Pipettenspitzen 2 an den Anwender oder Benutzer geliefert wird.

Je nach Verwendung und Einsatz können einzelne oder mehrere der Pipettenspitzen 2 entweder manuell oder in einem automatisierten Entnahmevorgang mittels eines nicht dargestellten Pipettors bei geöffnetem Deckel 4 entnommen werden. So dient bei diesem Ausführungsbeispiel die Aufnahmeschale 3 grundsätzlich dazu, den Träger 5 mitsamt den darauf angeordneten bzw. aufgenommenen Pipettenspitzen 2 positioniert für die Entnahme aufzunehmen. Sind die Pipettenspitzen 2 vom Träger 5 vollständig entnommen, wird der geleerte Träger 5 von der Aufnahmeschale 3 entfernt und es kann ein neuer mit Pipettenspitzen 2 bestückter Träger 5 wiederum in die Aufnahmeöffnung 12 der Aufnahmeschale 3 eingesetzt werden.

Somit umfasst der Pipettenspitzen-Aufnahmebehälter 1 in seiner Grundform die Aufnahmeschale 3 sowie den darauf abnehmbar gehaltenen Deckel 4. Bevorzugt werden die Aufnahmeschale 3 und der Deckel 4 getrennt voneinander hergestellt und können für die Auslieferung in eine zusammengebaute bzw. gefügte Stellung verbracht werden. Der Träger 5 mit den Pipettenspitzen 2 kann bereits aufgenommen sein oder ist dieser nachträglich einzusetzen. In dieser sogenannten Verschlussstellung des Deckels 4 deckt dieser die Aufnahmeöffnung 12 der Aufnahmeschale 3 ab.

Für die manuelle Handhabung bzw. Entnahme der Pipettenspitzen 2 ist der Deckel 4 soweit zu öffnen oder von der Aufnahmeschale 3 abzunehmen, sodass ein ungehinderter Zugang zu den einzelnen Pipettenspitzen 2 ermöglicht wird.

Für die automatisierte Entnahme von Pipettenspitzen 2 oder zur Probenverarbeitung ist hier vorgesehen, dass der Deckel 4 nicht nur ein Verschlusselement für die Aufnahmeschale 3 bildet, sondern zusätzlich auch noch als Adapterelement für die Aufnahmeschale 3 dient. Dazu wird, wie zuvor beschrieben, im Deckelinnenraum 19 im Bereich seiner Deckelwand 14 und/oder an der vom Deckelinnenraum 19 abgewendeten Seite an der Deckelwand 14 die Positioniervorrichtung 25 mit mehreren, bevorzugt über den Umfang des Deckels 4, verteilt angeordneten Positionierelementen 26 vorgesehen. Darüber hinaus wird der Deckel 4 in seinem äußeren Umfangsbereich zumindest abschnittsweise mit der normierten Standfläche ausgebildet, wobei von der normierten Standfläche die Längenabmessung 23 und die im rechten Winkel dazu ausgerichtete Breitenabmessung 24 definiert wird.

Zur Bildung der Adapterstellung des Deckels 4 für die Aufnahmeschale 3 ist der Deckel 4 von der Aufnahmeschale 3 abzunehmen. Anschließend daran wird die Aufnahmeschale 3 mit ihrem Boden 7 am Deckel 4 abgestützt. Dazu kann die Aufnahmeschale 3 entweder in den Deckelinnenraum 19 des Deckels 4 eingesetzt oder an der vom Deckelinnenraum 19 abgewendeten Seite auf der Deckelwand 14 abgestellt werden. Mittels der zuvor beschriebenen Positionierelemente 26 wird nun die Aufnahmeschale 3 relativ bezüglich der durch den Deckel 4 definierten Standfläche in einer exakten Positionierstellung positioniert dazu ausgerichtet. Damit bildet der Deckel 4 in der zueinander positionierten Stellung für die Aufnahmeschale 3 die Adapterstellung für die automatisierte Probenverarbeitung oder die Entnahme einzelner der Pipettenspitzen 2 aus. Es könnte aber auch unabhängig davon befüllte Pipettenspitzen 2 in den Träger 5 eingesetzt werden.

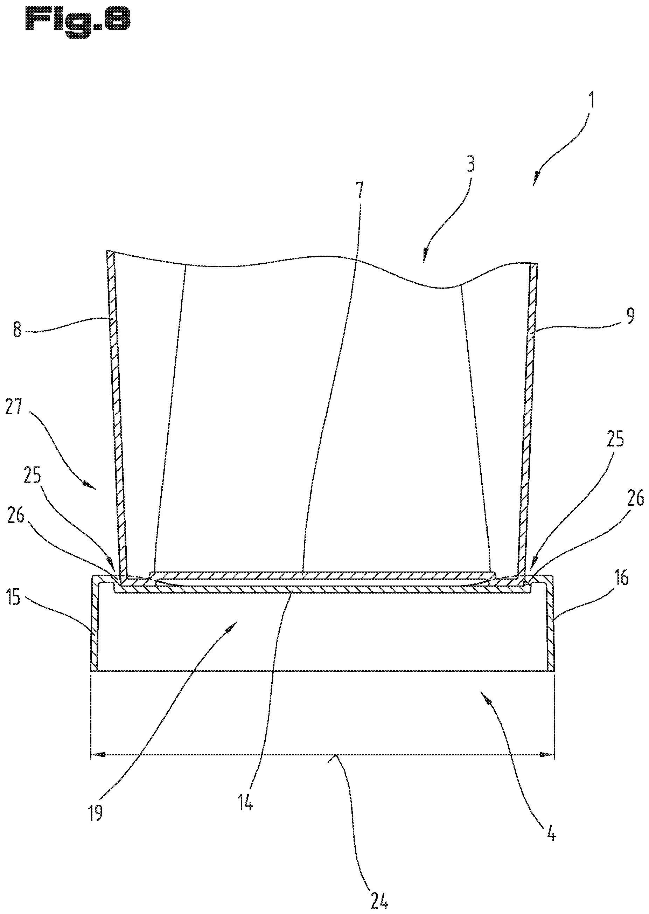

In der Fig. 8 ist eine weitere und gegebenenfalls für sich eigenständige Ausführungsform des Pipettenspitzen-Aufnahmebehälter 1 gezeigt, wobei wiederum für gleiche Teile gleiche Bezugszeichen bzw. Bauteilbezeichnungen wie in den vorangegangenen Fig. 1 bis 7 verwendet werden. Um unnötige Wiederholungen zu vermeiden, wird auf die detaillierte Beschreibung in den vorangegangenen Fig. 1 bis 7 hingewiesen bzw. Bezug genommen.

Da diese Ausbildung eine mögliche Alternative zu jener in der Fig. 5 bereits näher beschriebenen Anordnung darstellt, werden hier nur mehr die Unterschiede dazu näher beschrieben.

Der Deckel 4 ist auch hier als Adapterelement für die Aufnahmeschale 3 ausgebildet. Die Positioniervorrichtung 25 ist im Gegensatz zu der zuvor beschriebenen Ausführung nicht im Bereich des Deckelinnenraums 19, sondern an der vom Deckelinnenraum 19 abgewendeten Seite an der Deckelwand 14 angeordnet oder ausgebildet. Damit dient die offene Seite des Deckels 4 - also die Deckelseitenwände 15 bis 18 - als Standfläche in der positionierten Adapterstellung. Damit ist der Deckel 4 zumindest in diesem Abschnitt in seinen Abmessungen mit der normierten Standfläche auszubilden, wie dies zuvor bereits beschrieben worden ist.

Die Positionierelemente 26 können durch entsprechende Ausbildung der Deckelwand 14 selbst und/oder durch einen oder mehrere Stege oder Rippen gebildet sein. Im vorliegenden Ausführungsbeispiel könnten die Positionierelemente 26 auch gleichzeitig als Ausricht- bzw. Stapelhilfen für die Stapelbildung von mehreren übereinander gestapelten Pipettenspitzen-Aufnahmebehältern 1 dienen. Für die Stapelbildung von mehreren übereinander gestapelten Pipettenspitzen-Aufnahmebehältern 1 war es bereits bekannt, z.B. den äußeren Umfangsrand des Deckels 4 etwas über die Deckelwand 14 vorragen zu lassen, wobei aufgrund der großzügigen Toleranzen, jedoch keine exakte Positionierung zueinander möglich gewesen ist. Außerdem wies der Deckel auch nicht die normierte Standfläche auf.

Weiters wäre es aber auch noch möglich, sowohl die zuvor in den Fig. 1 bis 6 beschriebene Positioniervorrichtung 25 im Bereich des Deckelinnenraums 19 als auch die hier in der Fig. 8 beschriebene Anordnung derselben gleichzeitig an einem Deckel 4 auszubilden oder anzuordnen. Damit kann ein noch universellerer Einsatz des Deckels 4 erreicht werden.

Die Ausführungsbeispiele zeigen mögliche Ausführungsvarianten sowie Verwendungsbeispiele des Pipettenspitzen-Aufnahmebehälters 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus des Pipettenspitzen-Aufnahmebehälters 1 dieser bzw. dessen Bestandteile teilweise unmaßstäblich und/oder vergrößert und/oder verkleinert dargestellt wurden.

**Bezugszeichenaufstellung**

| | | | |
|---|---|---|---|
| 1 | Pipettenspitzen-Aufnahmebehälter | 31 | erstes Verschlusselement |
| 2 | Pipettenspitze | 32 | zweites Verschlusselement |
| 3 | Aufnahmeschale | | |
| 4 | Deckel | | |
| 5 | Träger | | |
| 6 | Zentrieraufnahme | | |
| 7 | Boden | | |
| 8 | Seitenwand | | |
| 9 | Seitenwand | | |
| 10 | Seitenwand | | |
| 11 | Seitenwand | | |
| 12 | Aufnahmeöffnung | | |
| 13 | Aufnahmeraum | | |
| 14 | Deckelwand | | |
| 15 | Deckelseitenwand | | |
| 16 | Deckelseitenwand | | |
| 17 | Deckelseitenwand | | |
| 18 | Deckelseitenwand | | |
| 19 | Deckelinnenraum | | |
| 20 | Schwenkanordnung | | |
| 21 | Schwenkzapfen | | |
| 22 | Schwenkauge | | |
| 23 | Längenabmessung | | |
| 24 | Breitenabmessung | | |
| 25 | Positioniervorrichtung | | |
| 26 | Positionierelement | | |
| 27 | Übergangsbereich | | |
| 28 | erstes Stapelmittel | | |
| 29 | weiteres Stapelmittel | | |
| 30 | Verschlussvorrichtung | | |

## Patentansprüche

1. Pipettenspitzen-Aufnahmebehälter (1) zur Aufnahme einer Mehrzahl von Pipettenspitzen (2), umfassend
- eine Aufnahmeschale (3) mit einem Boden (7) und mit vom Boden (7) aufragenden Seitenwänden (8 bis 11), welche Seitenwände (8 bis 11) zumindest bereichsweise eine Aufnahmeöffnung (12) umgrenzen sowie gemeinsam mit dem Boden (7) einen Aufnahmeraum (13) definieren;
- einen Deckel (4) mit einer Deckelwand (14) und mit von der Deckelwand (14) abstehenden Deckelseitenwänden (15 bis 18), welche Deckelseitenwände (15 bis 18) gemeinsam mit der Deckelwand (14) einen Deckelinnenraum (19) definieren;
- wobei der Deckel (4) in seiner Verschlussstellung die Aufnahmeöffnung (12) der Aufnahmeschale (3) abdeckt und abnehmbar an der Aufnahmeschale (3) gehalten ist,
**dadurch gekennzeichnet,**
- **dass** der Deckel (4) in seinem äußeren Umfangsbereich zumindest abschnittsweise eine gemäß der Norm SLAS 1-2004 (R2012) des American National Standards Instituts (ANSI) normierte Standfläche mit einer Längenabmessung (23) mit einem Wert von 127,76 mm ± 0,25 mm und mit einer im rechten Winkel dazu ausgerichteten Breitenabmessung (24) mit einem Wert von 85,48 mm ± 0,25 mm definiert,
- **dass** am Deckel (4) eine Positioniervorrichtung (25) mit mehreren, insbesondere über den Umfang des Deckels (4), verteilt angeordneten Positionierelementen (26) vorgesehen ist,
- **dass** der Deckel (4) bei einer von der Aufnahmeschale (3) abgenommenen Freigabestellung ein Adapterelement für die Aufnahmeschale (3) bildet, und
- **dass** die Aufnahmeschale (3) in der abgenommenen Freigabestellung des Deckels (4) mit ihrem Boden (7) an dem von der Aufnahmeschale (3) abgenommenen Deckel (4) zur Bildung einer Adapterstellung abgestützt ist, und in dieser Adapterstellung die Aufnahmeschale (3) mittels der Positionierelemente (26) relativ bezüglich der durch den Deckel (4) definierten Standfläche in einer Positionierstellung für eine automatisierte Probenverarbeitung positioniert ausgerichtet ist.

2. Pipettenspitzen-Aufnahmebehälter (1) nach Anspruch 1, **dadurch gekennzeichnet, dass** die Positioniervorrichtung (25) im Deckelinnenraum (19) des Deckels (4) sowie im Bereich seiner Deckelwand (14) angeordnet oder ausgebildet ist.

3. Pipettenspitzen-Aufnahmebehälter (1) nach Anspruch 1 oder 2, **dadurch gekennzeichnet, dass** die Positioniervorrichtung (25) an der Deckelwand (14) sowie an seiner vom Deckelinnenraum (19) abgewendeten Seite angeordnet oder ausgebildet ist.

4. Pipettenspitzen-Aufnahmebehälter (1) nach einem der vorhergehenden Ansprüche, **dadurch gekennzeichnet, dass** die Aufnahmeschale (3) in deren Positionierstellung direkt an der Deckelwand (14) abgestützt ist.

5. Pipettenspitzen-Aufnahmebehälter (1) nach einem der vorhergehenden Ansprüche, **dadurch gekennzeichnet, dass** die Aufnahmeschale (3) jeweils in Übergangsbereichen (27) zwischen den in Umfangsrichtung hintereinander angeordneten Seitenwänden (8 bis 11) an diesen sowie in deren dem Boden (7) zugewendeten Umfangsbereich an den Positionierelementen (26) abgestützt ist.

6. Pipettenspitzen-Aufnahmebehälter (1) nach Anspruch 5, **dadurch gekennzeichnet, dass** am Deckel (4), insbesondere an dessen Deckelwand (14), für jeden der Übergangsbereiche (27) der Aufnahmeschale (3) zwei Positionierelemente (26) vorgesehen sind.

7. Pipettenspitzen-Aufnahmebehälter (1) nach einem der vorhergehenden Ansprüche, **dadurch gekennzeichnet, dass** bei sich in der Positionierstellung befindlichen Aufhahmeschale (3) diese zentrisch bezüglich der vom Deckel (4) definierten Standfläche ausgerichtet ist.

8. Pipettenspitzen-Aufnahmebehälter (1) nach einem der vorhergehenden Ansprüche, **dadurch gekennzeichnet, dass** der Deckel (4) mittels zumindest einer Schwenkanordnung (20) an der Aufnahmeschale (3) schwenkbar gelagert ist.

9. Pipettenspitzen-Aufnahmebehälter (1) nach einem der vorhergehenden Ansprüche, **dadurch gekennzeichnet, dass** die Aufnahmeschale (3) im Bereich ihrer Seitenwände (8 bis 11) einschalig ausgebildet ist.

10. Pipettenspitzen-Aufnahmebehälter (1) nach einem der vorhergehenden Ansprüche, **dadurch gekennzeichnet, dass** die Seitenwände (8 bis 11) der Aufnahmeschale (3) im Axialschnitt gesehen jeweils ausgehend von der Aufnahmeöffnung (12) hin in Richtung auf den Boden (7) konisch verjüngend ausgebildet sind und dass die Aufnahmeschale (3) im Bereich ihrer Aufnahmeöffnung (12) eine lichte Querschnittsabmessung aufweist, welche größer ausgebildet ist als eine äußere Querschnittsabmessung der Aufnahmeschale (3) im Bereich des Bodens (7).

11. Pipettenspitzen-Aufnahmebehälter (1) nach einem der vorhergehenden Ansprüche, **dadurch gekennzeichnet, dass** an der Aufnahmeschale (3) im Bereich ihres Aufnahmeraums (13) zumindest ein erstes Stapelmittel (28) angeordnet oder ausgebildet ist, und eine weitere baugleich ausgebildete Aufnahmeschale (3) in den Aufnahmeraum (13) einsetzbar und an dem zumindest einen ersten Stapelmittel (28) abstützbar ist.

12. Pipettenspitzen-Aufnahmebehälter (1) nach Anspruch 11, **dadurch gekennzeichnet, dass** der Boden (7) der Aufnahmeschale (3) zumindest bereichsweise ein weiteres Stapelmittel (29) bildet.

13. Pipettenspitzen-Aufnahmebehälter (1) nach einem der vorhergehenden Ansprüche, **dadurch gekennzeichnet, dass** in der Aufnahmeschale (3) im Bereich ihrer Aufnahmeöffnung (12) ein Träger (5) mit darin angeordneten Zentrieraufnahmen (6) zur ausgerichteten Aufnahme der Pipettenspitzen (2) angeordnet oder ausgebildet ist.

14. Pipettenspitzen-Aufnahmebehälter (1) nach einem der vorhergehenden Ansprüche, **dadurch gekennzeichnet, dass** der Deckel (4) in seiner Verschlussstellung mittels zumindest einer Verschlussvorrichtung (30) an der Aufnahmeschale (3) arretiert gehalten ist, wobei die zumindest eine Verschlussvorrichtung (30) am Deckel (4) ein erstes Verschlusselement (31) und an der Aufnahmeschale (3) ein damit zusammenwirkendes zweites Verschlusselement (32) umfasst.

15. Pipettenspitzen-Aufnahmebehälter (1) nach Anspruch 14, **dadurch gekennzeichnet, dass** das am Deckel (4), insbesondere an zumindest einer seiner Deckelseitenwände (15 bis 18), angeordnete erste Verschlusselement (31) lappenförmig ausgebildet ist und schwenkbar mit dem Deckel (4) verbunden ist, und dass bei sich in der Positionierstellung befindlicher Aufnahmeschale (3) das erste Verschlusselement (31) in den Deckelinnenraum (19) eingeschwenkt ist und an der unmittelbar benachbart angeordneten Seitenwand (8 bis 11) der Aufnahmeschale (3) anliegt.

16. Verfahren zum Bereitstellen eines Pipettenspitzen-Aufhahmebehälters (1) zur Aufnahme einer Mehrzahl von Pipettenspitzen (2), bei dem
- eine Aufnahmeschale (3) mit einem Boden (7) und mit vom Boden (7) aufragenden Seitenwänden (8 bis 11) ausgebildet wird, wobei von den Seitenwänden (8 bis 11) zumindest bereichsweise eine Aufnahmeöffnung (12) umgrenzt sowie gemeinsam mit dem Boden (7) ein Aufnahmeraum (13) definiert wird;
- ein Deckel (4) mit einer Deckelwand (14) und mit von der Deckelwand (14) abstehenden Deckelseitenwänden (15 bis 18) ausgebildet wird, wobei von den Deckelseitenwänden (15 bis 18) gemeinsam mit der Deckelwand (14) ein Deckelinnenraum (19) definiert wird;
- und in einer Verschlussstellung des Deckels (4) von diesem die Aufnahmeöffnung (12) der Aufnahmeschale (3) abdeckt wird und der Deckel (4) an der Aufnahmeschale (3) abnehmbar gehalten wird und der Deckel (4) nach der Abnahme von der Aufnahmeschale (3) in eine Freigabestellung verbringbar ist;
**dadurch gekennzeichnet,**
- **dass** der Deckel (4) in seinem äußeren Umfangsbereich zumindest abschnittsweise mit einer gemäß der Norm SLAS 1-2004 (R2012) des American National Standards Instituts (ANSI) normierten Standfläche ausgebildet wird und von der normierten Standfläche eine Längenabmessung (23) mit einem Wert von 127,76 mm ± 0,25 mm und eine im rechten Winkel dazu ausgerichtete Breitenabmessung (24) mit einem Wert von 85,48 mm ± 0,25 mm definiert wird,
- **dass** am Deckel (4) eine Positioniervorrichtung (25) mit mehreren, insbesondere über den Umfang des Deckels (4), verteilt angeordneten Positionierelementen (26) vorgesehen wird,
- **dass** der Deckel (4) von der Aufnahmeschale (3) abgenommen und in seine Freigabestellung verbracht wird und anschließend die Aufnahmeschale (3) mit ihrem Boden (7) am Deckel (4) abgestützt wird, und dabei vom Deckel (4) ein Adapterelement zur Bildung einer Adapterstellung für die Aufnahmeschale (3) ausgebildet wird,
- **dass** die Aufnahmeschale (3) mittels der Positionierelemente (26) relativ bezüglich der durch den Deckel (4) definierten Standfläche in der Adapterstellung positioniert ausgerichtet wird, und
- **dass** in der Adapterstellung vom Deckel (4) in der zueinander positionierten Stellung für die Aufnahmeschale (3) eine Positionierstellung für eine automatisierte Probenverarbeitung ausgebildet wird.

17. Verfahren nach Anspruch 16, **dadurch gekennzeichnet, dass** die Aufnahmeschale (3) in deren Positionierstellung am Deckel (4) zentrisch bezüglich der vom Deckel (4) definierten Standfläche ausgerichtet wird.

18. Verfahren nach Anspruch 16 oder 17, **dadurch gekennzeichnet, dass** die Aufnahmeschale (3) in deren Positionierstellung im Deckel (4) zusätzlich mit einer in etwa parallel bezüglich der Deckelwand (14) ausgerichteten Stellkraft auf zumindest eine ihrer Seitenwände (8 bis 11) wirkend beaufschlagt wird und die Aufnahmeschale (3) in Wirkungsrichtung der Stellkraft von dieser gegen zumindest einzelne der Positionierelemente (26) gedrückt wird.

## Claims

1. Pipette tip-holding container (1) for accommodating a plurality of pipette tips (2), comprising
- a receiving cradle (3) having a base (7) and side walls (8 to 11) which project upwards from the base (7), which side walls (8 to 11) surround at least a portion of a receiving opening (12) and together with the base (7) define a receiving area (13);
- a cover (4) having a cover wall (14) and having cover side walls (15 to 18) which project from the cover wall (14), which cover side walls (15 to 18) together with the cover wall (14) define a cover interior (19);
- wherein in its closed position the cover (4) covers the receiving opening (12) of the receiving cradle (3) and is held on the receiving cradle (3) so as to be removable, **characterized in that**
- at least a portion of the outer peripheral region of the cover (4) defines a footprint which is standardized according to standard SLAS 1-2004 (R2012) of the American National Standards Institute (ANSI) with a length dimension (23) having a value of 127.76 mm ± 0.25 mm and with a width dimension (24) aligned at right angles thereto having a value of 85.48 mm ± 0.25 mm,
- a positioning device (25) with multiple positioning elements (26) which are arranged particularly in distribution over the periphery of the cover (4) is provided on the cover (4),
- in an open position when removed from the receiving cradle (3), the cover (4) forms an adapter element for the receiving cradle (3), and
- in the open position with the cover (4) removed, the receiving cradle (3) is supported by its base (7) on the cover (4) which has been removed from the receiving cradle (3) to create an adapter position, and in said adapter position the receiving cradle (3) is aligned by means of the positioning elements (26) relatively with reference to the footprint defined by the cover (4) in a positioning position for automated sample processing.

2. Pipette tip-holding container (1) according to claim 1, **characterized in that** the positioning device (25) is arranged or formed in the cover interior (19) of the cover (4) and in the region of the cover wall (14) thereof.

3. Pipette tip-holding container (1) according to claim 1 or 2, **characterized in that** the positioning device (25) is arranged or formed on the cover wall (14) and on the side facing away from the cover interior (19).

4. Pipette tip-holding container (1) according to any one of the preceding claims, **characterized in that** the receiving cradle (3) is supported directly on the cover wall (14) in the positioning position thereof.

5. Pipette tip-holding container (1) according to any one of the preceding claims, **characterized in that** the receiving cradle (3) is supported in each case in transition regions (27) between the side walls (8 to 11), which are arranged in circumferential direction one behind another, on said transition regions (27) and in their peripheral region facing the base (7) against said positioning elements (26).

6. Pipette tip-holding container (1) according to claim 5, **characterized in that** two positioning elements (26) are provided on the cover (4), in particular on the cover wall (14) thereof, for each of the transition regions (27) of the receiving cradle (3).

7. Pipette tip-holding container (1) according to any one of the preceding claims, **characterized in that** when the receiving cradle (3) is situated in the positioning position, it is aligned centrally with reference to the footprint defined by the cover (4).

8. Pipette tip-holding container (1) according to any one of the preceding claims, **characterized in that** the cover (4) is mounted pivotably on the receiving cradle (3) by means of at least one pivoting arrangement (20).

9. Pipette tip-holding container (1) according to any one of the preceding claims, **characterized in that** the receiving cradle (3) is constructed in a single-walled manner in the region of its side walls (8 to 11).

10. Pipette tip-holding container (1) according to any one of the preceding claims, **characterized in that** the side walls (8 to 11) of the receiving cradle (3) are constructed in conically tapering manner viewed in axial cross-section, beginning in each case from the receiving opening (12) towards the base (7), and **in that** the receiving cradle (3) has a clear cross-sectional dimension in the region of its receiving opening (12) which is designed to be greater than an outer cross-sectional dimension of the receiving cradle (3) in the region of the base (7).

11. Pipette tip-holding container (1) according to any one of the preceding claims, **characterized in that** at least one first stacking means (28) is arranged or formed on the receiving cradle (3) in the region of the receiving area (13) thereof, and a further structurally identical receiving cradle (3) is insertable into the receiving area (13) and is supportable on the at least one first stacking means (28).

12. Pipette tip-holding container (1) according to claim 11, **characterized in that** at least a region of the base (7) of the receiving cradle (3) forms a further stacking means (29).

13. Pipette tip-holding container (1) according to any one of the preceding claims, **characterized in that** a carrier (5) with centring receiving means (6) arranged therein for the aligned receiving of the pipette tips (2) is arranged or formed in the receiving cradle (3) in the region of the receiving opening (12) thereof.

14. Pipette tip-holding container (1) according to any one of the preceding claims, **characterized in that** in its closed position the cover (4) is held in a locked manner on the receiving cradle (3) by means of at least one closing device (30), wherein the at least one closing device (30) comprises a first closure element (31) on the cover (4) and a second closure element (32) which cooperates therewith on the receiving cradle (3).

15. Pipette tip-holding container (1) according to claim 14, **characterized in that** the first closure element (31), which is arranged on the cover (4), in particular on at least one of the cover side walls (15 to 18), is constructed in a lobed manner and is connected to the cover (4) so as to be pivotable, and **in that**, with the receiving cradle (3) situated in the positioning position the first closure element (31) is pivoted into the cover interior (19) and abuts against the side wall (8 to 11) of the receiving cradle (3) which is arranged directly adjacent thereto.

16. Method for providing a pipette tip-holding container (1) for accommodating a plurality of pipette tips (2), in which
- a receiving cradle (3) is realized with a base (7) and with side walls (8 to 11) which project up from the base (7), wherein the side walls (8 to 11) surround a receiving opening (12) at least in regions and define a receiving area (13) together with the base (7);
- a cover (4) is realized with a cover wall (14) and with cover side walls (15 to 18) which protrude from the cover wall (14), wherein the cover side walls (15 to 18) together with the cover wall (14) define a cover interior (19);
- and with the cover (4) in a closed position the receiving opening (12) of the receiving cradle (3) is covered by said cover and the cover (4) is held detachably on the receiving cradle (3) and the cover (4) is movable into an open position after detachment from the receiving cradle (3);
**characterized in that**
- at least a part of the outer peripheral region of the cover (4) is constructed with a footprint which is standardized according to standard SLAS 1-2004 (R2012) of the American National Standards Institute (ANSI) and a length dimension (23) with a value of 127.76 mm ± 0.25 mm and a width dimension (24) aligned at right angles thereto with a value of 85.48 mm ± 0.25 mm are defined by the standardized footprint,
- a positioning device (25) having multiple positioning elements (26) which are arranged particularly in distribution over the periphery of the cover (4) is provided on the cover (4),
- the cover (4) is removed from the receiving cradle (3) and moved into its open position, and the receiving cradle (3) is then supported by its base (7) on the cover (4), and in this way an adapter element is formed by the cover (4) for creating an adapter position for the receiving cradle (3),
- the receiving cradle (3) is aligned in position with respect to the footprint defined by the cover (4) in position in the adapter position by means of the positioning elements (26), and
- with the cover (4) in the adapter position a positioning position is created in the relatively positioned position for the receiving cradle (3) for automated sample processing.

17. Method according to claim 16, **characterized in that** in its positioning position on the cover (4) the receiving cradle (3) is aligned centrally with reference to the footprint defined by the cover (4).

18. Method according to claim 16 or 17, **characterized in that** in its positioning position on the cover (4) at least one of the side walls (8 to 11) of the receiving cradle (3) is/are further acted upon by an actuating force that is directed approximately parallel with reference to the cover wall (14), and the receiving cradle (3) is pressed in the effective direction of the actuating force by said force against at least individual positioning elements of the positioning elements (26).

## Revendications

1. Récipient de logement de pointes de pipettes (1) pour le logement d'une pluralité de pointes de pipettes (2), comprenant :
- une coque de logement (3) avec un fond (7) et des parois latérales (8 à 11) s'élevant à partir du fond (7), ces parois latérales (8 à 11) entourant au moins à certains endroits une ouverture de logement (12) et définissant, conjointement avec le fond (7), un espace de logement (13) ;
- un couvercle (4) avec une paroi de couvercle (14) et des parois latérales de couvercle (15 à 18) dépassant de la paroi du couvercle (14), ces parois latérales de couvercle (15 à 18) définissant, conjointement avec la paroi du couvercle (14), un espace interne de couvercle (19) ;
- le couvercle (4) recouvrant, dans sa position fermée, l'ouverture de logement (12) de la coque de logement (3) et étant maintenu de manière amovible sur la coque de logement (3),
**caractérisé en ce que**
- le couvercle (4) définit, sur sa partie circonférentielle externe, au moins à certains endroits, une surface d'appui normalisée selon la norme SLAS 1-2004 (R2012) de l'American National Standard Institute (ANSI), avec une dimension longitudinale (23) avec une valeur de 127,76 ± 0,25 mm et avec une dimension en largeur (24) orientée à angle droit par rapport à celle-ci, avec une valeur de 85,48 ± 0,25 mm,
- sur le couvercle (4), est prévu un dispositif de positionnement (25) avec plusieurs éléments de positionnement (26) disposés de manière répartie, plus particulièrement sur la circonférence du couvercle (4),
- le couvercle (4) forme, dans une position de déverrouillage retirée de la coque de logement (3), un élément adaptateur pour la coque de logement (3) et
- la coque de logement (3) est appuyée, dans la position de déverrouillage du couvercle (4), avec son fond (7), contre le couvercle (4) retiré de la coque de logement (3), afin de former une position d'adaptation, et, dans cette position d'adaptation, la coque de logement (3) est orientée, de manière positionnée au moyen des éléments de positionnement (26), par rapport à la surface d'appui définie par le couvercle (4), dans une position de positionnement pour un traitement d'échantillon automatisé.

2. Récipient de logement de pointes de pipettes (1) selon la revendication 1, **caractérisé en ce que** le dispositif de positionnement (25) est disposé ou réalisé dans l'espace interne (19) du couvercle (4) ainsi qu'au niveau de sa paroi de couvercle (14).

3. Récipient de logement de pointes de pipettes (1) selon la revendication 1 ou 2, **caractérisé en ce que** le dispositif de positionnement (25) est disposé ou réalisé sur la paroi du couvercle (14) ainsi qu'au niveau de son côté opposé à l'espace interne du couvercle (19).

4. Récipient de logement de pointes de pipettes (1) selon l'une des revendications précédentes, **caractérisé en ce que** la coque de logement (3) est appuyée, dans sa position de positionnement, directement contre la paroi du couvercle (14).

5. Récipient de logement de pointes de pipettes (1) selon l'une des revendications précédentes, **caractérisé en ce que** la coque de logement (3) est appuyée, dans des zones de transition (27) respectives entre les parois latérales (8 à 11) disposées les unes derrière les autres dans la direction de la circonférence, contre celles-ci ainsi que, dans la partie de la circonférence orientée vers le fond (7), contre les éléments de positionnement (26).

6. Récipient de logement de pointes de pipettes (1) selon la revendication 5, **caractérisé en ce que**, sur le couvercle (4), plus particulièrement sur sa paroi (14), pour chacune des zones de transition (27) de la coque de logement (3), sont prévus deux éléments de positionnement (26).

7. Récipient de logement de pointes de pipettes (1) selon l'une des revendications précédentes, **caractérisé en ce que**, lorsque la coque de logement (3) se trouve dans la position de positionnement, celle-ci est orientée de manière centrée par rapport à la surface d'appui définie par le couvercle (4).

8. Récipient de logement de pointes de pipettes (1) selon l'une des revendications précédentes, **caractérisé en ce que** le couvercle (4) est logé de manière pivotante au niveau de la coque de logement (3) au moyen d'un dispositif de pivotement (20).

9. Récipient de logement de pointes de pipettes (1) selon l'une des revendications précédentes, **caractérisé en ce que** la coque de logement (3) est réalisée sous la forme d'une seule coque au niveau de ses parois latérales (8 à 11).

10. Récipient de logement de pointes de pipettes (1) selon l'une des revendications précédentes, **caractérisé en ce que** les parois latérales (8 à 11) de la coque de logement (3), vues en coupe axiale, sont réalisées sous une forme conique se rétrécissant à partir de l'ouverture de logement (12) en direction du fond (7) et **en ce que** la coque de logement (3) présente, au niveau de son ouverture de logement (12), une dimension de section transversale intérieure qui est supérieure à une dimension de section transversale extérieure de la coque de logement (3) au niveau du fond (7).

11. Récipient de logement de pointes de pipettes (1) selon l'une des revendications précédentes, **caractérisé en ce que**, sur la coque de logement (3), au niveau de son espace de logement (13) est disposé ou réalisé au moins un premier moyen d'empilement (28) et une autre coque de logement (3), construite de manière identique, peut être insérée dans l'espace de logement (13) et peut être appuyée contre l'au moins un premier moyen d'empilement (28).

12. Récipient de logement de pointes de pipettes (1) selon la revendication 11, **caractérisé en ce que** le fond (7) de la coque de logement (3) forme, au moins à certains endroits, un autre moyen d'empilement (29).

13. Récipient de logement de pointes de pipettes (1) selon l'une des revendications précédentes, **caractérisé en ce que**, dans la coque de logement (3), au niveau de son ouverture de logement (12), est disposé ou réalisé un support (5) avec des logements de centrage (6) disposés à l'intérieur, pour le logement orienté des pointes de pipettes (2).

14. Récipient de logement de pointes de pipettes (1) selon l'une des revendications précédentes, **caractérisé en ce que** le couvercle (4) est maintenu bloqué sur la coque de logement (3), dans sa position fermée, au moyen d'au moins un dispositif de fermeture (30), l'au moins un dispositif de fermeture (30) comprenant sur le couvercle (4) un premier élément de fermeture (31) et sur la coque de logement (3) un deuxième élément de fermeture (32) interagissant avec celui-ci.

15. Récipient de logement de pointes de pipettes (1) selon la revendication 14, **caractérisé en ce que** le premier élément de fermeture (31), disposé sur le couvercle, plus particulièrement sur au moins une de ses parois latérales (15 à 18), est conçu avec des lobes et est relié de manière pivotante avec le couvercle (4), et **en ce que**, lorsque la coque de logement (3) se trouve dans la position de positionnement, le premier élément de fermeture (31) est replié dans l'espace interne du couvercle (19) et s'appuie contre la paroi latérale (8 à 11), disposée à proximité immédiate, de la coque de logement (3).

16. Procédé de fabrication d'un récipient de logement de pointes de pipettes (1) pour le logement d'une pluralité de pointes de pipettes (2), dans lequel
- une coque de logement (3) est réalisée avec un fond (7) et avec des parois latérales (8 à 11) s'élevant du fond (7), une ouverture de logement (12) étant entourée, au moins à certains endroits, par les parois latérales (8 à 11) et un espace de logement (13) étant défini conjointement avec le fond (7) ;
- un couvercle (4) est réalisé avec une paroi de couvercle (14) et avec des parois latérales (15 à 18) s'élevant à partir de la paroi du couvercle (14), un espace interne de couvercle (19) étant défini par les parois latérales du couvercle (15 à 18) conjointement avec la paroi du couvercle (14) ;
- et dans une position de fermeture du couvercle (4), l'ouverture de logement (12) de la coque de logement (3) est recouverte par celui-ci et le couvercle est maintenu de manière amovible sur la coque de logement (3) et le couvercle (4) peut être amené, après le retrait de la coque de logement (3) dans une position de déverrouillage ;
**caractérisé en ce que**
- le couvercle (4) est réalisé, sur sa partie circonférentielle externe, au moins à certains endroits, avec une surface d'appui normalisée selon la norme SLAS 1-2004 (R2012) de l'American National Standard Institute (ANSI), et la surface d'appui définit une dimension longitudinale (23) avec une valeur de 127,76 ± 0,25 mm et une dimension en largeur (24) orientée à angle droit par rapport à celle-ci, avec une valeur de 85,48 ± 0,25 mm,
- sur le couvercle (4), est prévu un dispositif de positionnement (25) avec plusieurs éléments de positionnement (26) disposés de manière répartie, plus particulièrement sur la circonférence du couvercle (4),
- le couvercle (4) est retiré de la coque de logement (3) et est amené dans sa position de déverrouillage puis la coque de logement (3) est appuyée, avec son fond (7), contre le couvercle (4) et un élément adaptateur est formé par le couvercle (4) pour la formation d'une position d'adaptation pour la coque de logement (3),
- la coque de logement (3) est orientée de manière positionnée, au moyen des éléments de positionnement (26), dans la position d'adaptation, par rapport à la surface d'appui définie par le couvercle (4) et
- dans la position d'adaptation, le couvercle (4) adopte, dans la position positionnée entre elles pour la coque de logement (3), une position de positionnement pour un traitement d'échantillon automatisé.

17. Procédé selon la revendication 16, **caractérisé en ce que** la coque de logement (3) est orientée, sans sa position de positionnement sur le couvercle (4) de manière centrée par rapport à la surface d'appui définie par le couvercle (4).

18. Procédé selon la revendication 16 ou 17, **caractérisé en ce que** la coque de logement (3) est en outre sollicitée, dans sa position de positionnement dans le couvercle (4), avec une force de déplacement orientée de manière approximativement parallèle à la paroi du couvercle (14), sur au moins une de ses parois latérales (8 à 11) et la coque de logement (3) est comprimée dans la direction d'action de la force de déplacement par celle-ci contre au moins certains des éléments de positionnement (26).
